# FASCICULE DE BREVET EUROPEEN

(11) **EP 2 282 650 B1**
(45) Date de publication et mention de la délivrance du brevet: **22.05.2013**
(21) Numéro de dépôt: 09745991.1
(22) Date de dépôt: 24.04.2009
(51) Int. Cl.: A41B 11/12, A61F 13/08

(54) **BANDE AUTO-FIXANTE DE MAINTIEN ET DE CONTENTION NOTAMMENT POUR BAS DE CONTENTION**
SELBSTKLEBENDES HALTE- UND KOMPRESSIONSBAND, INSBESONDERE FÜR EINEN KOMPRESSIONSSTRUMPF
SELF-ATTACHING TAPE FOR RETAINING AND COMPRESSION, PARTICULARLY FOR COMPRESSION STOCKING

(30) Priorité: 30.04.2008 FR 0852909
(43) Date de publication de la demande: 16.02.2011
(73) Titulaire: SIGVARIS AG, 9014 St. Gallen (CH)
(72) Inventeur: MATHIEU, Florence, 42380 Estivareilles (FR); ROMUALD, Gaëtan, 69500 Bron (FR)
(74) Mandataire: Dupuis, François
(86) Numéro de dépôt international: PCT/FR2009/050760
(87) Numéro de publication internationale: WO 2009/138672

(56) Documents cités:
- EP-A- 1 079 017
- WO-A-98/02120
- WO-A-2004/082935
- FR-A- 1 540 295
- FR-A- 2 887 124

## Description

L'invention se rattache au secteur technique des bandes de maintien et de contention, et aussi des bas, chaussettes de contention et pour compression utilisant une extrémité supérieure ou en débordement, avec des enductions en contact avec la peau du porteur.

Ce concept, dans son principe, est connu depuis de nombreuses années, en particulier pour des bas en assurant un maintien autour de la cuisse ou mollet du porteur, selon la destination.

De très nombreux brevets ont été déposés par de nombreux fabricants avec, à l'origine, le brevet français FR 1.540.295, et bien d'autres ensuite.

L'utilisation d'enduction siliconée est ainsi connue pour des applications de bas, chaussettes, à usage normal, et aussi de contention, compression et maintien.

Les produits existant sur le marché présentent, selon les cas, un certain nombre d'inconvénients au porter et à l'enlèvement.

Un premier inconvénient réside dans le fait que l'effet de contention sur le membre porteur, entraîne, même lorsqu'il n'y a pas de phénomène de striction, du fait du contact et des mouvements du porteur, des frottements, des effets de cisaillement gui génèrent des irritations désagréables et, à terme, douloureuses. Ceci est dû à la texture du support et à une certaine rigidité de l'enduction.

Même lorsque la peau n'est pas particulièrement sensible aux agents chimiques ou allergisants, elle risque d'être irritée par le port d'une bande auto-fixante si deux ou trois conditions suivantes sont réunies : prolifération bactérienne, frottement, macération.

S'agissant des frottements, l'irritation est du type mécanique due à la différence de comportement mécanique entre le support et son induction et la peau.

Le risque d'irritation augmente avec l'âge (peau plus fine et plus fragile). Or, il est indispensable de protéger la peau de la personne plus âgée car elle se régénère plus lentement.

Le risque augmente également :
- avec la chaleur, l'été en particulier, avec le phénomène de sudation ;
- lors d'une activité physique intensive, mouvement et sudation.

De la macération résulte de la fragilisation de la peau (moindre résistance mécanique) et augmentation de la perméabilité, donc moindre résistance aux agents pathogènes et irritants chimiques. Cette macération est provoquée par un manque d'évaporation de la vapeur d'eau (perspiration) naturellement émise par la peau, et il y a donc une nécessité que le produit soit perméable à la vapeur d'eau.

Un autre inconvénient rencontré, lors du port d'une bande auto-fixante, est celui de la pilosité des membres sur lesquels il peut être enfilé car s'il y a alors des frottements, les irritations et l'inconfort seront d'autant plus grands. Le maintien du bas ou de la bande, par une fixation présentant une forte adhésivité (type sparadrap), rendrait leur enlèvement également très douloureux par phénomène d'arrachage.

On connaît par ailleurs par le brevet EP 1 079 017 une bande auto-fixante ou complexe du type comprenant une structure textile (collant) avec une enduction pour assurer le maintien sur la peau d'un porteur, la partie support de l'enduction étant établie sous la forme d'un support tricoté ou tissé, susceptible d'être extensible, comprenant au moins sur sa surface intérieure une enduction ayant des propriétés adhérentes immédiates et importantes, l'enduction étant appliquée à une couche mince, sur la partie support elle-même fine et aérée (partie supérieure du collant).

Cette bande auto-fixante telle que définie dans ce brevet EP 1 079 017 ne permet pas de résoudre la problématique liée à l'irritation provoquée par les frottements de la bande auto-fixante sur la peau.

C'est à partir de ce constat général que le demandeur a recherché un nouveau type de bande auto-fixante permettant de supprimer les frottements et de réduire considérablement le risque de macération et donc d'irritation cutanée.

A titre complémentaire aussi, un autre but recherché était d'améliorer le confort au porter et les conditions d'enlèvement de la bande auto-fixante ou bas en réduisant les effets induits, en particulier du type tirage des poils.

Une autre démarche du demandeur était donc d'améliorer le confort au porter avec un produit léger, c'est-à-dire d'aspect visuel plus fin que les produits sur le marché.

Il fallait donc concevoir une nouvelle bande auto-fixante offrant une compression maîtrisée, une tenue optimisée en réduisant les différents risques et situations favorisant les irritations cutanées.

Or, de nombreuses recherches ont été menées avec des tests de contrôle pour améliorer tel ou tel paramètre.

En pratique, ce type d'expérimentation ne s'est pas avéré concluant à la connaissance du déposant.

Selon l'invention, une démarche globale a été menée, tant dans le choix du support que de l'enduction, permettant la conception d'une bande auto-fixante permettant d'avoir une optimisation des effets rendus par une sélection très particulière de ses composants, apportant des effets indirects inattendus au porter et à l'enlèvement en cas de forte pilosité du porteur.

Selon une première caractéristique de l'invention, la bande auto-fixante ou complexe est du type comprenant une structure textile avec une enduction de silicone pour assurer le maintien sur la peau d'un porteur, la bande étant caractérisée en ce que la partie support de l'enduction est établie sous la forme d'un support tricoté ou tissé, susceptible d'être extensible, qui comprend au moins sur sa surface intérieure une enduction ayant des propriétés de tack immédiates et importantes, et en ce que l'enduction est définie avec un degré de mollesse pour se conformer aux microreliefs du substrat tel que la peau du porteur et en ce que le module élastique de l'enduction est défini pour être au moins aussi bas que celui du substrat contre lequel il est en contact pour épouser les micro-déformations de celui-ci et en ce que l'enduction est appliquée à une couche mince, sur la partie support elle-même fine et aérée.

Selon une autre caractéristique, la partie support de l'enduction est établie sous la forme d'une dentelle.

Ainsi, la bande auto-fixante, selon l'invention, définit un complexe extensible élastique ayant une adhésivité et des propriétés physiques ou chimiques et mécaniques particulières permettant d'avoir une compression maîtrisée et une tenue optimisée sur le membre en réduisant ou supprimant le risque des irritations cutanées.

Ainsi, selon l'invention, cette bande auto-fixante peut s'appliquer sur des bas et chaussettes de contention ou autre article de contention et de compression du type bandage.

Ces caractéristiques et d'autres encore ressortiront bien de la suite de la description.

Pour fixer l'objet de l'invention illustrée aux figures des dessins où :
- la figure 1 est une vue partielle d'une bande auto-fxante, selon l'invention, dans sa version simple face ;
- la figure 2 est une vue partielle de la bande auto-fixante dans sa version double face ;
- la figure 3 est une vue partielle à grande échelle montrant l'un des intérêts de l'invention dans la configuration où l'enduction épouse étroitement des formes apparentes rugueuses du substrat, lorsque celui-ci est la peau d'un porteur, par exemple ;
- la figure 4 est une vue d'un exemple d'application de la bande auto-fixante selon l'invention dans sa version simple face et appliquée à un bas médical de contention et/ou de compression ;
- la figure 5 est une vue d'un exemple d'application de la bande auto-fixante dans sa version double face, avec un dispositif de compression et/ou de contention ;
- la figure 6 est un exemple de l'application de la bande auto-fixante dans une version simple face en étant disposée dans un dispositif de compression et/ou de contention ;
- la figure 7 est un exemple d'application de la bande auto-fixante dans une configuration d'enroulement.

Afin de rendre plus concret l'objet de l'invention, on le décrit maintenant d'une manière non limitative illustrée aux figures des dessins.

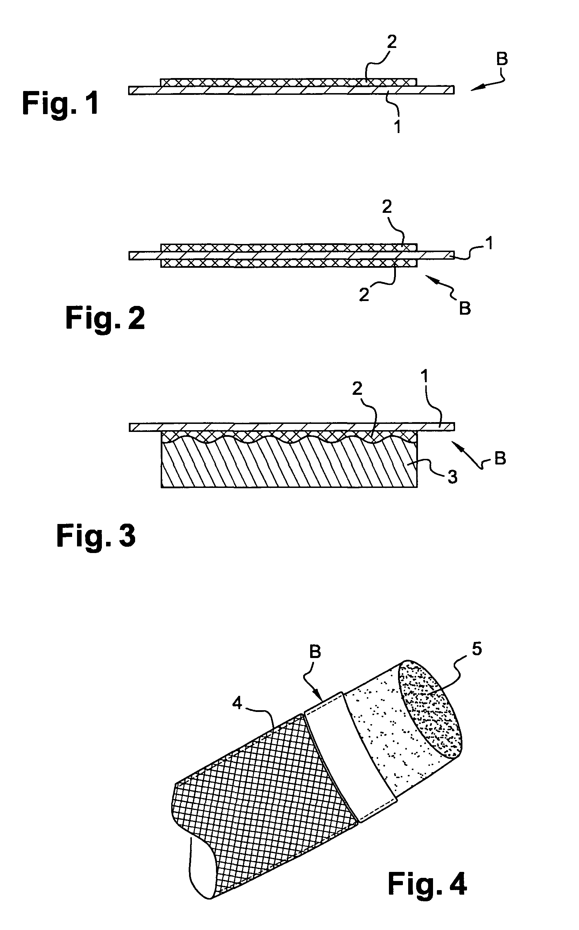

La bande auto-fixante (B) du complexe comprend une structure textile (1), constituant le support, et établie sous la forme d'un support tricoté ou tissé ayant des propriétés physiques (élasticité, extensibilité, état de surface, rugosité, épaisseur et perméabilité). Ce support, selon les applications envisagées, par exemple comme bas de contention ou de maintien, est réalisé sous la forme d'une dentelle. Cette structure textile, comme représentée aux figures 1 et 2 des dessins, peut être réalisée sur une forme simple face ou double face et inclut au moins, sur sa surface intérieure, et aussi, en variante, sur ses deux faces intérieure et extérieure, une enduction (2) ayant des propriétés de tack immédiates et importantes. Plus particulièrement, la ou les couches adhérentes obtenues par enduction ont une épaisseur de l'ordre de 0.5 mm. Les propriétés particulières de cette enduction sont son adhésivité immédiate associée à sa mollesse, appelées, dans les termes techniques professionnels, « tack », sa visco-élasticité, son taux de transmission de vapeur d'eau et sa bio compatibilité. Cette enduction sur la ou les faces intérieure et extérieure de la structure textile est définie avec un degré de mollesse pour se conformer et épouser les microreliefs du substrat (3) tel que la peau du porteur. Par ailleurs, le module élastique de l'enduction est défini pour être au moins aussi bas que celui du substrat contre lequel il est en contact pour épouser les micro-déformations de celui-ci.

L'enduction peut être mono ou multi-composants. Elle présente, selon l'invention, un fort tack et est constitué d'un adhésif de nature hydrophobe sensible à la pression. Les propriétés de tack, énergie et force de tack, sont mesurées par un test de probe tack et une analyse mécanique dynamique sur DMA Q800. Cet adhésif peut être du type polyuréthane, silicone, Styrène-Isopréne-Styrène et Styrène-Butadiène-Styrène. Dans un exemple avantageux, l'enduction met en oeuvre deux composants silicone développés par la Société MOMENTIVE sous la référence RTV 833, avec la mise en oeuvre d'un premier composant RTV 833A, soit un polydiméthylsiloxanes avec groupes vinyle et catalyseur platine, et un second composant RTV 833B, avec un mélange de polydiméthylsiloxanes, de mastiques et de réticulant. Le caractère mou est mesuré par pression. L'adhésivité de l'enduction est établie par grammage de l'enduction et la proportion et dosages des composants, une ou des résines adhésivantes pouvant être rapportées. Le silicone et le polyuréthane sont avantageusement utilisés du fait de leur haute perméabilité à la vapeur d'eau.

L'enduction est de préférence réalisée sous forme de film afin d'avoir une répartition la plus homogène possible sur le support. Sans sortir du cadre de l'invention, elle peut être établie selon une configuration continue ou discontinue. La bande auto-fixante ou complexe obtenue est de faible épaisseur, de l'ordre de 1 mm et l'enduction de 0.5 mm. L'épaisseur de l'enduction peut varier. Le grammage de l'enduction est inférieur à 500 g/m². De par la porosité de la structure textile constitutive du support pour faciliter la prise de l'enduction, le taux de transmission de vapeur d'eau est supérieur d'au moins 30 % à celle des bandes auto-fixantes classiques, et ceci du fait essentiellement du choix de la nature du support et de la réduction d'épaisseur de l'enduction.

La bande auto-fixante mise en oeuvre selon l'invention a fait l'objet de différents tests comparatifs par rapport à des productions antérieures du déposant, le produit de ce dernier étant identifié sous la marque SIGVARIS, et de produits de tiers fabricants. La base de comparaison et de test est le prototype F5001 du demandeur correspondant aux normes requises pour faire les mesures.

Le tableau ci-après définit l'extensibilité des bandes auto-fixantes. On remarque que toutes les bandes auto-fixantes actuellement sur le marché sont de 40 à 110 % plus fortes en compression pour 30 % d'allongement par rapport au prototype « F5001», base de la référence.

| Nom du produit | Fabricant | Code ACt. | Taille | Classe de compression | Largeur dentelle (cm) | Dim. à plat dentelle (cm) | Dim. dentelle 30% (cm) | Compression à 30% d'allgl (hPa) | Comp. à 30% d'allgl (hPa/cm) | Differentiel SIGVARIS F 5001 |
|---|---|---|---|---|---|---|---|---|---|---|
| Mediven Elegance Autolix Platinium | Medi | 4654052 | Small Long | 2 | 5,4 | 19.7 | 25,61 | 18,21 | 3,37 | 116.79 |
| Mediven Seduction | Medi | 4466617 | Small Court | 1 | 5,4 | 19.3 | 25,09 | 12,07 | 2,24 | 43,09 |
| Mediven Seduction | Innothers | 4458925 | T1 Normal | 2 | 5,6 | 19.5 | 25,35 | 13,02 | 1.97 | 55.00 |
| Vorismo Rellets de teint | Gibaud | 4592322 | 1 | 2 | 5,5 | 16.7 | 24,31 | 12.1 | 2.20 | 44.05 |
| Venoctil Secrel | Thusane | 4347611 | IN | 2 | 6,7 | 18.1 | 23,53 | 16 | 2.39 | 00,40 |
| Voile Radiante | Cognon Morin | 4554958 | 1M | 2 | 4.8 | 19.1 | 24,83 | 15,14 | 3,15 | 50,24 |
| Veinoslim Déesie | Pierre Fabre | 4536826 | T1 Normal | 2 | 6 | 17.5 | 22.75 | 12,70 | 2,13 | 52,20 |
| F 5001 | Sigvaris | TR 125-1 | SN | 2 | 5.4 | 20,6 | 20,78 | 8.4 | 1.56 | 0,00 |
| Nely | Sigvaris | | SL | 2 | 5.2 | 20.7 | 26.91 | 5.83 | 1.31 | -18,69 |
| Diaphono | Sigvaris | 7974314 | SN | 2 | 5,6 | 20,8 | 27,04 | 12,36 | 2,21 | 47,14 |

On a représenté, aux figures des dessins, la bande auto-fixante dans sa version simple face (figure 1) en contact avec le substrat ou peau et double face (figure 2).

Selon la figure 4, la bande auto-fixante qui est réalisée à partir d'une structure textile réalisée en dentelle se prolonge de la partie bas (4) médical ou manchon enfilé sur un membre (5) du porteur.

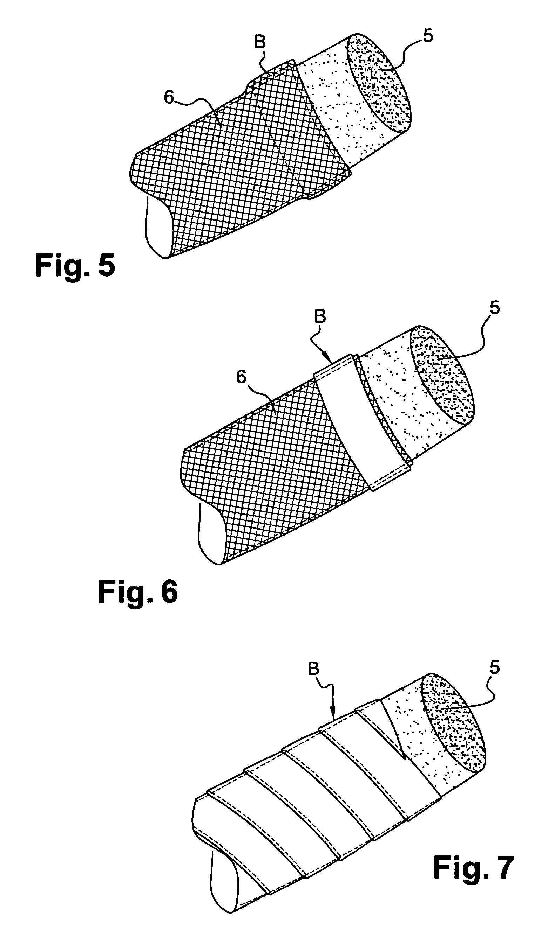

Selon la figure 5, la bande auto-fixante est double face et s'adapte sur un dispositif de compression / contention (6).

Selon la figure 6, la bande auto-fixante de l'invention s'adapte sur un dispositif de compression / contention (6) tout en laissant apparaître une bordure débordante de ce dernier.

Selon la figure 7, la bande auto-fixante simple face constitue elle-même un bandage enroulé en spires pour le chevauchement partiel.

Sans sortir du cadre de l'invention, l'enduction peut incorporer des substances hydrophiles sous forme de charges (hydrocolloides, pectine, ...) ou des micro capsules à relargage contrôlé permettant d'apporter une fonctionnalité ou traitement complémentaire (bactériostatique, bactéricide, anti-inflammatoire, cicatrisant, hydratant...).

La bande auto-fixante, selon l'invention, offre une optimisation de confort et de tenue, de par ses caractéristiques particulières en supprimant ou réduisant toutes les contraintes initiales identifiées générant des douleurs, des irritations sur la peau.

La capacité adhésive de l'enduction est suffisante pour que la bande auto-fixante tienne seule en place sans autre moyen.

Le retrait facile de la bande auto-fixante est sans douleur, sans résidus, est effectif aussi en situation de forte pilosité. Ce retrait facile est mesuré par un test de pelage à 180° réalisé sur substrat verre selon la norme NF EN 28510-2 après 24 h de tenue. L'enduction ayant un fort tack ne présente pas une force de pelage élevée. Cette dernière est sensiblement équivalente à celle d'une enduction classique, et reste en tout état de cause très faible, < 0,6 N/nm.

La bande auto-fixante, de par la combinaison de ses caractéristiques, permet d'éviter les frottements source d'irritation, en permettant d'épouser les microdéformations de la peau liées à son relief et à son extensibilité propre, et les macros déformations liées aux mouvements corporels. L'absence de résidus au retrait est liée à la cohésion de l'enduction vérifiée lors du test de pelage à 180°. Le probe test permet de déterminer l'énergie et la force du tack, qui pour l'enduction objet de l'invention sont telles que l'énergie de tack > 3.8N.nm (< 3.0 pour enduction classique) et la force de tack >8N (contre 4N pour une enduction classique). La DMA (analyse mécanique dynamique) permet de déterminer les modules G' et G" de façon corrélée avec les propriétés de tack. Les pentes sont nettement plus élevées sur l'enduction objet de l'invention :
- pente log G' >= 0.2 (contre 0.05 pour enduction classique),
- pente log G" >= 0.24 (contre 0.15 pour enduction classique).

## Revendications

1. Bande auto-fixante ou complexe du type comprenant une structure textile (1) avec une enduction pour assurer le maintien sur la peau d'un porteur, **caractérisée en ce que** la partie support de l'enduction (2) est établie sous la forme d'un support tricoté ou tissé, susceptible d'être extensible, qui comprend au moins sur sa surface intérieure une enduction (2) ayant des propriétés adhérentes immédiates et importantes,
et **en ce que** l'enduction est définie avec un degré de mollesse pour se conformer aux microreliefs du substrat (3) tel que la peau du porteur,
et **en ce que** le module élastique de l'enduction est défini pour être au moins aussi bas que celui du substrat contre lequel il est en contact pour épouser les micro-déformations de celui-ci,
et **en ce que** l'enduction est appliquée à une couche mince, sur la partie support elle-même fine et aérée.

2. Bande auto-fixante, selon la revendication 1, **caractérisée en ce que** la partie support de l'enduction est établie sous la forme d'une dentelle.

3. Bande auto-fixante, selon la revendication 1, **caractérisée en ce qu'**elle comprend une enduction simple face ou double face, mono ou muti-composants, avec une épaisseur de l'ordre de 0.5 mm.

4. Bande auto-fixante, selon la revendication 3, **caractérisée en ce que** l'enduction bi-composants met en oeuvre deux composants silicone avec un premier composant RTV 833A, soit un polydiméthylsiloxanes avec groupes vinyle et catalyseur platine, et un second composant RTV 833B, avec un mélange de polydiméthylsiloxanes, de mastiques et de réticulant.

5. Bande auto-fixante, selon la revendication 3, **caractérisée en ce que** l'enduction est à base de silicone ou de polyuréthanne, ou de Styrène-Isoprène-Styrène ou de Styrène-Butadiène-Styrène.

6. Bande auto-fixante, selon la revendication 3, **caractérisée en ce que** l'enduction présente une énergie de tack d'au moins 3.8 N.mm et une force de tack d'au moins 8N.

7. Bande auto-fixante, selon la revendication 3, **caractérisée en ce que** les modules G' et G" définis en analyse mécanique dynamique de façon corrélée avec les propriétés de tack sont plus élevées sur l'enduction avec pente log G' >= 0.2 et pente log G" >= 0.24.

8. Bande auto-fixante, selon la revendication 1, **caractérisée en ce que** la bande auto-fixante ou complexe obtenue est de faible épaisseur, de l'ordre de 1 mm et l'enduction de 0.5 mm,
et **en ce que** le grammage de l'enduction est inférieur à 500 g/m²,
et **en ce que** de par la porosité de la structure textile (1) constitutive du support pour faciliter la prise de l'enduction, le taux de transmission de vapeur d'eau ou perméabilité est supérieur de 30 % à celle des bandes auto-fixantes classiques utilisées.

## Patentansprüche

1. Selbstfixierendes Band bzw. Komplex nach Art derer mit einer textilen Struktur (1) mit einer Beschichtung für den sicheren Halt auf der Haut eines Trägers, **dadurch gekennzeichnet, dass** der die Beschichtung tragende Teil (2) aus einer dehnbaren gestrickten oder gewobenen Unterlage besteht, die mindestens auf ihrer Innenfläche eine Beschichtung (2) mit unmittelbaren und starken Hafteigenschaften besitzt,
und dass die Beschichtung mit einem Weichheitsgrad definiert ist, um sich an die Mikroreliefs des Substrats (3) - wie die Haut des Trägers - anzupassen,
und dass das Elastizitätsmodul der Beschichtung so definiert ist, um mindestens so niedrig zu sein wie dasjenige des Substrats, mit dem es in Kontakt ist, um sich genau an dessen Mikrodeformationen anzuschmiegen,
und dass die Beschichtung in einer dünnen Schicht auf den seinerseits feinen und luftigen Trägerteil aufgebracht wird.

2. Selbstfixierendes Band nach Anspruch 1, **dadurch gekennzeichnet, dass** der tragende Teil der Beschichtung als Spitze ausgeführt ist.

3. Selbstfixierendes Band nach Anspruch 1, **dadurch gekennzeichnet, dass** es eine einseitige oder doppelseitige Beschichtung aus einer oder mehreren Komponenten mit einer Dicke von etwa 0,5 mm umfasst.

4. Selbstfixierendes Band nach Anspruch 3, **dadurch gekennzeichnet, dass** bei der Zweikomponentenbeschichtung zwei Silikonkomponenten zum Einsatz kommen: eine erste Komponente RTV 833A, d.h. ein Polydimethylsiloxan mit Vinylgruppen und Platinkatalysator, und eine zweite Komponente RTV 833B mit einer Mischung aus Polydimethylsiloxanen, Mastixen und Vernetzer.

5. Selbstfixierendes Band nach Anspruch 3, **dadurch gekennzeichnet, dass** die Basis der Beschichtung Silikon oder Polyurethan oder Styrol-Isopren-Styrol oder Styrol-Butadien-Styrol ist.

6. Selbstfixierendes Band nach Anspruch 3, **dadurch gekennzeichnet, dass** die Beschichtung eine Tack-Energie von mindestens 3,8 N.mm und eine Tack-Kraft von mindestens 8 N aufweist.

7. Selbstfixierendes Band nach Anspruch 3, **dadurch gekennzeichnet, dass** die Module G' und G", die in der dynamischen mechanischen Analyse in Korrelation mit den Tack-Eigenschaften definiert werden, auf der Beschichtung mit Steigung log G' >= 0,2 und Steigung log G" >= 0,24 größer sind.

8. Selbstfixierendes Band nach Anspruch 1, **dadurch gekennzeichnet, dass** das erzielte selbstfixierende bzw. komplexe Band eine geringere Dicke in der Größenordnung von 1 mm und die Beschichtung eine Dicke von 0,5 mm aufweist,
und dass die Massenbelegung der Beschichtung geringer als 500 g/m² ist, und dass die Wasserdampftransmissionsrate oder Durchlässigkeit aufgrund der Porosität der - zum besseren Halt der Beschichtung - die Unterlage bildenden textilen Struktur (1) um 30 % höher ist als diejenige der herkömmlich verwendeten selbstfixierenden Bänder.

## Claims

1. A self-fixing tape or a complex of the type comprising a textile structure (1) with a coating for ensuring that it is held on the skin of a wearer, **characterised in that** the support part for the coating (2) is made in the form of a knitted or woven support, capable of being stretchable, which comprises at least on its inner surface a coating (2) having immediate and significant adhesive properties,
and **in that** the coating is defined with a degree of softness so as to accommodate to the microreliefs of the substrate (3) such as the wearer's skin,
and **in that** the modulus of elasticity of the coating is defined to be at least as low as that of the substrate with which it is in contact so as to follow the micro-deformations thereof,
and **in that** the coating is applied in a thin layer, on the support part which itself is fine and loosely woven.

2. A self-fixing tape according to claim 1, **characterised in that** the support part for the coating takes the form of lace.

3. A self-fixing tape according to claim 1, **characterised in that** it comprises a single-component or multicomponent coating on one or both sides, said coating having a thickness of the order of 0.5 mm.

4. A self-fixing tape according to claim 3, **characterised in that** the two-component coating makes use of two silicone components with a first component RTV 833A, i.e. a polydimethylsiloxane with vinyl groups and platinum catalyst, and a second component RTV 833B, with a mixture of polydimethylsiloxanes, fillers and cross-linking agent.

5. A self-fixing tape according to claim 3, **characterised in that** the coating is based on silicone or on polyurethane, or on styrene-isoprene-styrene or on styrene-butadiene-styrene.

6. A self-fixing tape according to claim 3, **characterised in that** the coating exhibits a tack energy of at least 3.8 N.mm and a tack force of at least 8 N.

7. A self-fixing tape according to claim 3, **characterised in that** the moduli G' and G" defined under dynamic mechanical analysis in correlation with the tack properties are higher over the coating with a gradient log G' ≥ 0.2 and a gradient log G" ≥ 0.24.

8. A self-fixing tape according to claim 1, **characterised in that** the self-fixing tape or complex obtained is of low thickness, of the order of 1 mm, while the coating is 0.5 mm thick,
and **in that** the weight per unit area of the coating is less than 500 g/m²,
and **in that**, due to the porosity of the textile structure (1) making up the support to facilitate the grab adhesion of the coating, the water vapour transmission rate or permeability is greater by 30% than that of conventional self-fixing tapes which are used.
